(19) Europäisches Patentamt
European Patent Office
Office européen des brevets

(11) Veröffentlichungsnummer: **0 258 733 B1**

(12) **EUROPÄISCHE PATENTSCHRIFT**

(45) Veröffentlichungstag der Patentschrift: **10.04.91**

(51) Int. Cl.⁵: **C07C 233/17**, C07C 233/60, A01N 37/20, A01N 53/00

(21) Anmeldenummer: **87111934.3**

(22) Anmeldetag: **18.08.87**

(54) **Carboxamide.**

(30) Priorität: **19.08.86 DE 3628082**

(43) Veröffentlichungstag der Anmeldung:
**09.03.88 Patentblatt 88/10**

(45) Bekanntmachung des Hinweises auf die Patenterteilung:
**10.04.91 Patentblatt 91/15**

(84) Benannte Vertragsstaaten:
**AT BE CH DE FR GB IT LI NL**

(56) Entgegenhaltungen:

**RESEARCH DISCLOSURE, Band 262, Februar 1986, Seite 103; "Ein Propionamid und dessen Herstellung."**

(73) Patentinhaber: **BASF Aktiengesellschaft**
**Carl-Bosch-Strasse 38**
**W-6700 Ludwigshafen(DE)**

(72) Erfinder: **Buerstinghaus, Rainer, Dr.**
**Roentgenstrasse 38**
**W-6900 Heidelberg(DE)**
Erfinder: **Hofmeister, Peter, Dr.**
**Bernard-Humblot-Strasse 12**
**W-6730 Neustadt(DE)**
Erfinder: **Kuenast, Christoph, Dr.**
**Muehlstrasse 21**
**W-6701 Waldse(DE)**

EP 0 258 733 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Carboxamide der allgemeinen Formel I

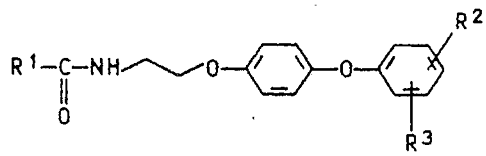

(I),

in der die Substituenten folgende Bedeutung haben:

- $R^1$ $C_1$-$C_6$-Alkyl, $C_2$-$C_6$-Alkenyl, $C_2$-$C_6$-Alkinyl, $C_3$-$C_{10}$-Cycloalkyl, $C_3$-$C_{10}$-Cycloalkenyl oder $C_4$-$C_{10}$-Cycloalkenyl-alkyl und
- $R^2$, $R^3$ Wasserstoff, Halogen, $C_1$-$C_3$-Alkyl, $C_1$-$C_3$-Alkoxy, $C_1$-$C_3$-Halogenalkyl, $C_1$-$C_3$-Halogenalkoxy oder Methylthio.

Außerdem betrifft die vorliegende Erfindung die Herstellung der Verbindungen I, Schädlingsbekämpfungsmittel, die die Verbindungen I enthalten, und ein Verfahren zur Bekämpfung von Schädlingen.

Aus der DE-A 26 33 069 ist der Cyclopropancarbonsäureester I'

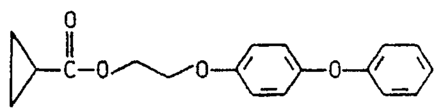

(I'),

aus der EP-A-4334 ist die Verbindung I"

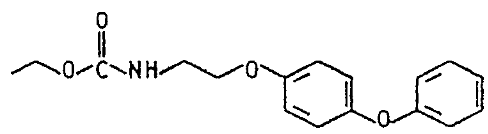

(I")

als Schädlingsbekämpfungsmittel bekannt.

Die Wirkung der Berbindungen I', I" läßt jedoch zu wünschen übrig.

Der Erfindung lag daher die Aufgabe zugrunde, neue Carboxamide I mit verbesserter Wirkung bereitzustellen.

Demgemäß wurden die eingangs definierten neuen Carboxamide I sowie Verfahren zu deren Herstellung gefunden. Ferner wurde gefunden, daß sich die Verbindungen I hervorragend zur Bekämpfung von Schädlingen eignen.

Die Verbindungen I sind aus 2-Phenoxy-ethylaminen II und Carbonsäurehalogeniden III erhältlich.

Die Umsetzung eines 2-Phenoxy-ethylamins II mit einem Carbonsäurehalogenid III, bevorzugt dem Säurechlorid von III,

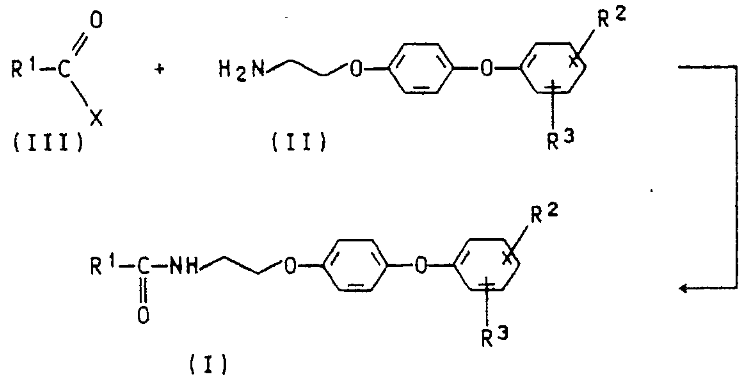

(III) (II) (I)

X = Fluor, Chlor, Brom oder Jod

führt in Anwesenheit eines säurebindenden Mittels bei Temperaturen von (-30) bis 120 °C, vorzugsweise von (-10) bis 80 °C und besonders bevorzugt von 0 bis 50 °C sowie Drücken zwischen 1 und 10 bar zu den Carboxamiden I. Als säurebindendes Mittel kann das 2-Phenoxy-ethylamin eingesetzt werden, üblicherwiese verwendet man jedoch die üblichen basischen Mittel, insbesondere aliphatische, aromatische oder heterocyclische Amine wie beispielsweise Triethylamin, Dimethylamin, Piperidin, Dimethylanilin, Dimethylbenzylamin, Pyridin und 4-Dimethylaminopyridin. Das Verhältnis säurebindendes Mittel zu Verbindung III beträgt 0,5:1 bis 20:1, vorzugsweise 0,7:1 bis 5:1 und besonders bevorzugt 0,9:1 bis 1,5:1.

Üblicherwiese setzt man die Ausgangsstoffe II und III in stöchiometrischem Verhältnis ein. Ein Überschuß der einen oder anderen Komponente kann in Einzelfällen aber durchaus vorteilhaft sein.

Die Umsetzung verläuft gewöhnlich oberhalb von (-30 °C) mit ausreichender Geschwindigkeit. 100 °C müssen im allgemeinen nicht überschritten werden. Da sie in einigen Fällen unter Wärmeentwicklung verläuft, kann es von Vorteil sein, eine Kühlmöglichkeit vorzusehen.

Die 2-Phenoxyethylamine II sind z.T. bekannt oder lassen sich nach bekannten Methoden (EP-A1-4334) herstellen. Die Carbonsäurehalogenide III sind bekannt und größenteils kommerziell erhältlich.

Die Umsetzungen werden zweckmäßigerwiese in einem Lösungs- oder Verdünnungsmittel ausgeführt. Hierzu sind beispielsweise geeignet: aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan und Chlorbenzol, Ether oder Ester wie Diethyl- und Di-n-Butylether, Methyl-tert.-butylether, Tetrahydrofuran, Dioxan und Essigsäureethylester; Ketone, beispielsweise Aceton, Methylethylketon und Methylisopropylketon; ferner Nitrile, wie Acetonitril und Propionitril; aprotische dipolare Lösungsmittel, wie Dimethylformamid, Dimethylsulfoxid oder Pyridin. Auch Gemische dieser Stoffe können als Lösungs- oder Verdünnungsmittel verwendet werden.

Die neuen Verbindungen der Formel I fallen teilweise in Form farbloser oder schwach bräunlich gefärbter Öle an, die sich durch längeres Erwärmen unter vermindertem Druck auf mäßig erhöhte Temperatur ("Andestillieren") von den letzten flüchtigen Anteilen befreien und auf diese Weise reinigen lassen. Erhält man die Verbindungen der Formel I in kristalliner Form, so kann ihre Reinigung durch Umkristallisation erfolgen.

Die erfindungsgemäßen Verbindungen der Formel I können außerdem noch nach praktisch allen bekannten Verfahren der Carboxamidsynthese hergestellt werden, beispielsweise durch Umsetzung von 2-Phenoxyethylaminen mit entsprechenden Carbonsäureestern, Carbonsäuren oder deren Salzen, Carbonsäureanhydriden oder Ketenderivaten (vgl. C. Ferri, Reaktionen der Organischen Synthese, Georg Thieme Verlag, Stuttgart 1978, S. 542 und die dort zitierte Literatur).

In den Verbindungen I haben die Substituenten folgende Bedeutung:

$R^1$- unverzweigtes oder verzweigtes $C_1$-$C_6$-Alkyl, bevorzugt $C_1$-$C_5$-Alkyl, wie Methyl und Ethyl, besonders bevorzugt $C_3$-$C_5$-Alkyl, wie n-Propyl, Isopropyl, n-Butyl, iso-Butyl, sec.-Butyl, tert.-Butyl, n-Pentyl, Isopentyl, 1-Methyl-butyl und 1,1-Dimethylpropyl,

- unverzweigtes und verzweigtes $C_2$-$C_6$-Alkenyl, bevorzugt $C_2$-$C_4$-Alkenyl, wie Allyl, 1-Methylallyl, Prop-1-en-1-yl und 2-Methylprop-1-en-1-yl,
- unverzweigtes und verzweigtes $C_2$-$C_6$-Alkinyl, bevorzugt $C_2$-$C_4$-Alkinyl, besonders bevorzugt Ethinyl,
- $C_3$-$C_{10}$-Cycloalkyl, bevorzugt $C_3$-$C_6$-Cycloalkyl, wie Cyclobutyl, Cyclopentyl und Cyclohexyl, besonders bevorzugt Cyclopropyl,
- $C_3$-$C_{10}$-Cycloalkenyl, bevorzugt $C_3$-$C_6$-Cycloalkenyl, wie Cyclopent-1-en-1-yl und Cyclohex-1-en-1-yl,
- $C_4$-$C_{10}$-Cycloalkenyl-alkyl, bevorzugt $C_4$-$C_8$-Cycloalkenyl-alkyl, wie Cyclopent-1-en-1-yl-methyl, 2-(Cyclopent-1-en-1-yl)-ethyl und Cyclohex-1-en-1-yl-methyl,

$R^2$, $R^3$ - Wasserstoff,

- Halogen, bevorzugt Fluor, Chlor und Brom,
- $C_1$-$C_3$-Alkyl, wie Methyl, Ethyl, n-Propyl und iso-Propyl,
- $C_1$-$C_3$-Alkoxy, wie Methoxy, Ethoxy, n-Propoxy und iso-Propoxy,
- $C_1$-$C_3$-Halogenalkyl, bevorzugt $C_1$-$C_2$-Fluoralkyl, wie Fluormethyl, Difluormethyl, Trifluormethyl und 2,2,2-Trifluoreth-1-yl,
- $C_1$-$C_3$-Halogenalkoxy, bevorzugt $C_1$-$C_2$-Fluoralkoxy, wie Fluormethoxy, Difluormethoxy und Trifluormethoxy,
- Methylthio.

Besonders bevorzugt sind Verbindungen I, in denen $R^2$ = $R^3$ = H ist.

Im Gegensatz zu den meisten bisher bekannten Wirkstoffen, die als Kontakt-oder Fraßgifte die Tiere töten, lähmen oder vertrieben, greifen die Verbindungen der Formel I in das hormonale System des tierischen Organismus ein. Bei Insekten wird beispielsweise die Umwandlung zur Imago, die Ablage von entwicklungsfähigen Eiern und die Entwicklung von abgelegten normalen Eiern gestört und dadurch die Generationsfolge unterbrochen. Für Wirbeltiere sind die erfindungsgemäßen Wirkstoffen praktisch ungiftig. Die Verbindungen der Formel I werden überdies leicht zu Stoffen abgebaut, die in der natürlichen Umgebung vorkommen und von Mikroorganismen weiter zerlegt werden. Die Gefahr einer Kumulation ist deshalb ausgeschlossen. Sie können demgemäß unbedenklich zur Bekämpfung von Schädlingen bei Tieren, Pflanzen und Vorräten und im Wasser eingesetzt werden.

Die Carboxamide der allgemeinen Formel I sind geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria mellonella (Wachsmotte), Malacosoma neustria (Ringelspinner), Dendrolimus pini (Kiefernspinner), Thaumatopea ptiyocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestis (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagai (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-puncta (Südlicher Maiswurzelwurm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Ortiorrhynchus sulcatus (Gefurchter Lappenrüßler), Otiorrhynchus ovatus (Erdbeerwurzelrüßler), Hylobius abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrucker), Blastophagus piniperda (Gefurchter Waldgärtner);

aus der Ordnung der Zweiflügler (Diptera) beispielsweise Lycoria pectoralis, Mayetiola destructor

(Hessenfliege), Basineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hysocyami (Rübenfliege), Anopheles maculipennis, Culex pipiens, Aedis aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege), Oestrus ovis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), Atta sexdens (Blattschneiderameise);
aus der Ordnung der Wanzen (Heteroptera) beispielsweise Nezara viridula (Grüne Reiswanze), Eurygaster integriceps (Asiatische Getreidewande), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielsweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlauf), Aphidula nasturtii (Kreuzdornblattlaus), Cerosipha gossypii (Gurkenblattlauf), Sappaphis mali (Rosige Apfellaus), Sappaphis mala (Mehlige Birnblattlaus), Dysaphis radicola (Mehige Apfelfaltenlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifungus, Calotermes flavicollis, Leucotermes flavipes, Termes natalensis;
aus der Ordnung der Geradflügler (Orthoptera) beispielsweise Forficula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melanoplus femur-rubrum (Rotbeinige Heuschrecke), Blatta orientalis (Küchenschabe), Blattella germanica (Deutsche Schabe), Periplaneta americana (Amerikanische Schabe), Blabera gigantes (Riesenschabe).

Zur Klasse der Arachnoidea gehören Spinnentiere (Acarina) beispielsweise Ixodes ricinus (Holzbock), Ornithodorus moubata, Amblyomma americanum, Dermacentor silvarum, Boophilus microplus, Tetranychus telarius, Tetranychus pacificus, Paratetranychus pilosus, Bryobia praetiosa.

Zur Klasse der Nematoden zählen beispielsweise Wurzelgallennematoden, z.B. Meloidogyne incognita, Meloidogyne hapla, Meloidogyne javanica, Zysten bildende Nematoden, z.B. Globodera rostochiensis, Heterodera schachtii, Heterodera avenae, Heterodera glycinae, Heterodera trifolii, Stock- und Blattälchen, z.B. Ditylenchus dipsaci, Ditylenchus destructor, Pratylenchus neglectus, Pratylenchus penetrans, Pratylenchus goodeyi, Paratylenchus curvitatus sowie Tylenchorhynchus dubius, Tylenchorhynchus claytoni, Rotylenchus robustus, Heliocotylenchus multicinctus, Radopholus similis, Belonolaimus longicaudatus, Longidorus elongatus, Trichodorus primitivus.

Die Wirkstoffe können als solche, in Form ihrer Formulierungen oder den daraus bereiteten Anwendungsmöglichkeiten, z.B. in Form direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate,

Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können als Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkali, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenon, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolylglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 5 Gew.-Teile der Verbindung Nr. 2 werden mit 95 Gew.-Teilen feinteiligem Kaolin innig vermischt. Man erhält auf diese Weise ein Stäubemittel, das 3 Gew.-% des Wirkstoffs enthält.

II. 30 Gew.-Teile der Verbindung Nr. 2 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gew.-Teile der Verbindung Nr. 2 werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht.

IV. 20 Gew.-Teile der Verbindung Nr. 2 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht.

V. 80 Gew.-Teile der Verbindung Nr. 2 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen.

Granulate z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magneisumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %. Die Wirkstoffe können auch mit gutem Erfolg im Ultra-Low-Volume-Verfahren (ULV) verwendet werden, wobei es möglich ist. Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,02 bis 10, vorzugsweise 0,05 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Beispielsweise können folgende Mittel zugemischt werden: 1,2-Dibrom-3-chlorpropan, 1,3-Dichlorpropen, 1,3-Dichlorpropen + 1,2-Dichlorpropan, 1,2-Dibromethan, 2-sec.-Butyl-phenyl-N-methylcarbamat, o-Chlorphenyl-N-methylcarbamat, 3-Isopropyl-5-methylphenyl-N-methylcarbamat, o-Isopropoxyphenyl-N-methylcarbamat, 3,5-Dimethyl-4-methylmercapto-phenyl-N-methylcarbamat, 4-Dimethylamino-3,5-xylyl-N-methylcarbamat, 2-(1,3-Dioxolan-2-yl)-phenyl-N-methylcarbamat, 1-Naphthyl-N-methylcarbamat, 2,3-Dihydro-2,2-dimethyl-benzofuran-7-yl-N-methylcarbamat, 2,2-Dimethyl-1,3-benzodioxol-4-yl-N-methylcarbamat, 2-Dimethylamino-5,6-dimethyl-4-pyrimidinyl-dimethylcarbamat, 2-Methyl-2-(methylthio)-propionaldehyd-O-(methylcarbamoyl)-oxim, S-Methyl-N[(methylcarbamoyl)-oxyl]-thio-acetimidat, Methyl-N',N'-dimethyl-N-[-(methylcarbamoyl)oxy]-1-thiooxamidat, N-(2-Methylchlorphenyl)-N',N'-dimethylformamidin, Tetrachlorthiophen, 1-(2,6-Difluor-benzoyl)-3-(4-chlorphenyl)-harnstoff, O,O-Dimethyl-O-(p-nitrophenyl)-phosphorthioat, O,O-Diethyl-O-(p-nitrophenyl)-phosphorthioat, O-Ethyl-O(p-nitrophenyl)-phenyl-phosphonothioat, O,O-Dimethyl-O-(3-methyl-4-nitrophenyl)-phosphorthioat, O,O-Diethyl-O-(2,4-dichlorphenyl)phosphorthioat, O-Ethyl-O-(2,4-dichlorphenyl)-phenyl-phosphonothioat, O,O-Dimethyl-O-(2,4,5-trichlorphenyl)-phosphorthioat, O-Ethyl-O-(2,4,5-trichlorphenyl)-ethyl-phosphorthioat, O,O-Dimethyl-O-(4-brom-2,5-dichlorphenyl)-phosphorthioat, O,O-Dimethyl-O-(2,5-dichlor-4-jodphenyl)-phosphorthioat, O,O-Dimethyl-O-(3-methyl-4-methylthiophenyl)-phosphorthioat, O-Ethyl-O-(3-methyl-4-methylthiophenyl)-isopropyl-phosphoramidat, O,O-Diethyl-O-(p-methylsulfinyl-phenyl)-phosphorthioat, O-Ethyl-S-phenylethyl-phosphonodithioat, O,O-Diethyl-[2-chlor-1-(2,4-dichlorphenyl)-vinyl]-phosphat, O,O-Dimethyl-[2-chlor-1-(2,4,5-trichlorphenyl)]-vinylphosphat, O,O-Dimethyl-S-(1'-phenyl)-ethylacetat-phosphordithioat, Bis-(dimethylamino)-fluorphosphinoxid, Octamethyl-pyrophosphoramid, O,O,O,O-Tetraethyldithio-pyrophosphat, S-Chlormethyl-O,O-diethyl-phosphordithioat, O-Ethyl-S,S-dipropyl-phosphordithioat, O-O-Dimethyl-O,2,2-dichlorvinyl-phosphat, O,O-Dimethyl-1,2-dibrom-2,2-dichlorethylphosphat, O,O-Dimethyl-2,2,2-trichlor-1-hydroxy-ethylphosphonat, O,O-Dimethyl-S-[1,2-biscarbethoxy-ethyl-(1)]-phosphordithioat, O,O-Dimethyl-O-(1-methyl-2-carbmethoxy-vinyl)-phosphat, O,O-Dimethyl-S-(N-methyl-carbamoylmethyl)-phosphordithioat, O,O-Dimethyl-S-(N-methylcarbamoyl-methyl)-phosphorthioat, O,O-Dimethyl-S-(N-methoxyethyl-carbamoylmethyl)-phosphordithioat, O,O-Dimethyl-S-(N-formyl-N-methyl-carbamoyl-methyl)-phosphordithioat, O,O-Dimethyl-O-[1-methyl-2-(methyl-carbamoyl)-vinyl]-phosphat, O,O-Dimethyl-O-[(1-methyl-2-dimethylcarbamoyl)-vinyl]-phosphat, O,O-Dimethyl-O-[(1-methyl-2-chlor-2-diethylcarbamoyl)-vinyl]-phosphat, O,O-Diethyl-S-(ethylthiomethyl)-phosphordithioat, O,O-Diethyl-S[(p-chlorphenylthio)-methyl]-phosphordithioat, O,O-Dimethyl-S-(2-ethylthioethyl)-phosphorthioat, O,O-Dimethyl-S-(2-ethylthioethyl)-phosphordithioat, O,O-Dimethyl-S-(2-ethylsulfinyl-ethyl)-phosphorthioat, O,O-Diethyl-S-(2-ethylthio-ethyl)-phosphordithioat, O,O-Diethyl-S-(2-ethylsulfinylethyl)-phosphorthioat, O,O-Diethyl-thiophosphoryliminophenyl)-acetonitril, O,O-Diethyl-S-(2-chlor-1-phthalimidoethyl)-phosphordithioat, O,O-Diethyl-S-[6-chlor-benzoxazolon-(2)-yl(3)]-methyldithiophosphat, O,O-Dimethyl-S-[2-methoxy-1,3,4-thiadiazol-5-[4H]-onyl-(4)-methyl]-phosphordithioat, O,O-Diethyl-O-[3,5,6-trichlor-pyridyl-(2)]-phosphorthioat, O,O-Diethyl-O-(2-pyrazinyl)-phosphorthioat, O,O-Diethyl-O-[2-isopropyl-4-methylpyrimidinyl(6)]-phosphorthioat, O,O-Diethyl-0-[2-(diethylamino)-6-methyl-4-pyrimidinyl]-thionophosphat, O,O-Dimethyl-S-(4-oxo-1,2,3-benzotriazin-3-[4H]-yl-methyl)-phosphordithioat, O,O-Dimethyl-S-[(4,6-diamino-1,3,5-triazin-2-yl)-methyl]-phosphordithioat, O,O-Diethyl-(1-phenyl-1,2,4-triazol-3-yl)-thionophosphat, O,S-Dimethyl-phosphor-amidothioat, O,S-Dimethyl-N-acetyl-phosphoramidothioat, alpha-Hexachlorcyclohexan, 1,1-Di-(p-methoxyphenyl)-2,2,2-trichlor-ethan, 6,7,8,9,10,10-Hexachloro-1,5,5a,6,9,9a-hexahydro-6,9-methano-2,4,3-benzodioxa-thiepin-3-oxid, Pyrethrine, DL-2-Allyl-3-methyl-cyclopenten-(2)-on-(1)-yl-(4)-DL-cis,-trans-chrysanthemat, 5-Benzyl-furyl-(3)-methyl-DL-cis,-trans-chrysanthemat, 3-Phenoxybenzyl(±)-cis,-trans-2,2-dimethyl-3-(2,2-dichlorvinyl)-cyclopropancarboxylat, alpha-Cyano-3-phenoxybenzyl(±)-cis,-trans-2,2-dimethyl-3-(2,2-dichlorvinyl)-cyclopropancarboxylat, (s)-alpha-Cyano-3-phenoxybenzyl-cis(1R,3R)-2,2-dimethyl-3-(2,2-dibromvinyl)-cyclopropancarboxylat, 3,4,5,6-Tetrahydrophthalimidoethyl-DL-cis,-trans-chrysanthemat, 2-Methyl-5-(2-propinyl)-3-furylmethyl-chrysanthemat, (alpha-Cyano-3-phenoxybenzyl)-alpha-isopropyl-4-chlorphenylacetat.

Herstellungsbeispiele

Beispiel 1

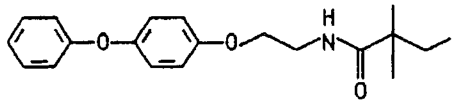

10,1 g 2-(4-Phenoxy-phenoxy)-ethylamin werden in 40 ml wasserfreiem Pyridin gelöst. Mit Hilfe eines Eisbades kühlt man unter Rühren auf 5° C ab. Anschließend läßt man eine Lösung von 2,2-Dimethylbuttersäurechlorid in 20 ml Methylenchlorid langsam hinzutropfen. Nach Beendigung der stark exothermen Reaktion wird 12 Stunden bei 25° C gerührt, in die dreifache Volumenmenge Wasser gegossen und viermal mit Methylenchlorid extrahiert. Der Extrakt wird gründlich mit Wasser gewaschen, über Natriumsulfat getrocknet und i.Vak. eingeengt. Den Rückstand nimmt man in Hexan/Methyl-tert.-butylether (4 : 1) auf und filtriert über Kieselgel -60 (Merck). Nach dem Abziehen des Solvens verbleibt ein Feststoff, der sich aus Hexan/Methyl-tert.-butylether umkristallisieren läßt. Beigefarbene Kristalle, Fp.: 47 - 48° C. Ausbeute: 8,8 g, 61 % der rechnerisch möglichen Menge.

$C_{20}H_{25}NO_3$ (327)

ber.: C 73,4 H 7,7 N 4,3

gef.: C 73,3 H 7,6 N 4,2

Infrarotabsorptionen ($cm^{-1}$): 1487, 1465, 1232, 819.

Beispiel 2

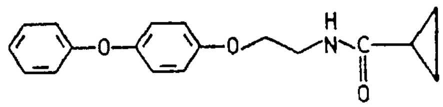

8,01 g 2-(4-Phenoxy-phenoxy)-ethylamin werden in 50 ml Essigsäureethylester gelöst und mit 5,6 g Pyridin versetzt. Unter Eiskühlung fügt man 3,65 g Cyclopropancarbonsäurechlorid in 25 ml Essigester tropfenweise hinzu. Nach dem Abklingen der Wärmeentwicklung wird noch 10 Stunden bei Raumtemperatur gerührt und filtriert. Die klare Lösung wird je dreimal mit Wasser, 5 %iger Salzsäure und erneut mit Wasser gewaschen, über Natriumsulfat getrocknet und im Rotationsverdampfer eingeengt. Man kristallisiert aus Petrolether/Essigester (3 : 1) um, wobei man 5,4 g eines fast farblosen Kristallpulvers mit einem Schmelzbereich von 101 bis 102° C erhält.

Ausbeute: 52 % der rechnerisch möglichen Menge.

$C_{18}H_{19}NO_3$ (297)

ber.: C 72,7 H 6,4 N 4,7

gef.: C 72,5 H 6,4 N 4,7

Infrarotabsorptionen ($cm^{-1}$): 1491, 1252, 1228, 1037, 841

Die in der nachstehenden Tabelle aufgeführten Verbindungen I wurden ebenfalls auf den in den Beispielen 1 und 2 beschriebenen Wegen erhalten, soweit sie durch ihre Absorptionsspektren identifiziert sind; andere Verbindungen, die der Formel (I) entsprechen, können auf die gleiche Weise erhalten werden.

| Bsp. Nr. | $R^1$ | $R^2$ | $R^3$ | X | Infrarotabsorption ($cm^{-1}$) |
|---|---|---|---|---|---|
| 3 | $n-C_3H_7$ | H | H | O | 1491, 1252, 1225, 1058, 843 |
| 4 | $i-C_3H_7$ | H | H | O | 1490, 1250, 1227, 842 |
| 5 | $CH_2-CH(CH_3)_2$ | H | H | O | 1492, 1260, 1239, 1224, 840 |
| 6 | tert.-$C_4H_9$ | H | H | O | 1489, 1229, 1222, 1060 |
| 7 | $-CH=CH_2$ | H | H | O | 1491, 1252, 1229, 842 |
| 8 | $-CH=CH-CH_3$ | H | H | O | 1491, 1252, 1225, 843 |
| 9 | $-CH=C(CH_3)_2$ | H | H | O | 1492, 1259, 1238, 839 |
| 10 | 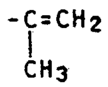 | H | H | O | 1491, 1252, 1233, 1218 |
| 11 | $-C≡C-H$ | H | H | O | |
| 15 | 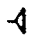 | 4-Cl | H | O | |
| 16 | 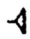 | 4-F | H | O | |
| 17 | 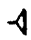 | 3-F | H | O | |
| 18 | 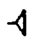 | 2-F | H | O | |
| 19 | 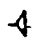 | 2-F | 4-F | O | |
| 20 | 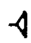 | 3-F | 5-F | O | |

Tabelle (Fortsetzung)

| Bsp. Nr. | $R^1$ | $R^2$ | $R^3$ | X | Infrarotabsorption ($cm^{-1}$) |
|---|---|---|---|---|---|
| 21 | 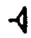 | 3-Cl | 5-Cl | O | |
| 22 | 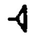 | $4-CH_3$ | H | O | |
| 23 | 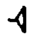 | $4-C_2H_5$ | H | O | |
| 24 | 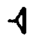 | $4-OCH_3$ | H | O | |
| 25 | 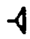 | $4-OC_2H_5$ | H | O | |
| 26 | 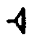 | $4-CH(CH_3)_2$ | H | O | |
| 27 | 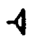 | 4-Br | H | O | |
| 28 | 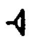 | $4-CF_3$ | H | O | |
| 29 | 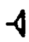 | $4-OCF_3$ | H | O | |
| 30 | $CH_3$ | H | H | O | |
| 31 | $C_2H_5$ | H | H | O | |
| 32 | 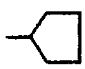 | H | H | O | |
| 33 | 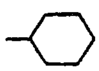 | H | H | O | 1492, 1260, 1239, 1211, 839 |
| 34 | 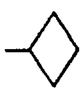 | H | H | O | 1491, 1251, 1227, 842 |
| 35 | $-CH_2$ 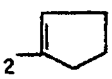 | H | H | O | |

Anwendungsbeispiele

In den folgenden Beispielen wurden die erfindungsgemäße Verbindung Nr. 2 hinsichtlich ihrer Wirkung auf Schädlinge gegen die nachstehende Verbindung des Standes der Technik verglichen:

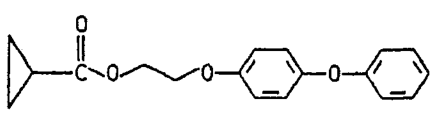

bekannt aus DE-A-26 33 069 in Beispiel 1

Beispiel A

Zuchtversuch mit Dysdercus indermedius (Baumwollwanzen)

1 l-Gläser werden mit 200 g sterilem Quarzsand gefüllt, der mit 25 ml der wäßrigen Wirkstoffaufbereitung gemischt ist. In jedes Gefäß werden 20 Larven im 4. Larvenstadium gesetzt, die mit gequollenem Baumwollsamen gefüttert werden.

Zum Zeitpunkt der Adulthäutung bei der Kontrollgruppe wird bonitiert, ob sich typische Juvenoideffekte zeigen, nämlich verkürzte oder gebogene Flügel. Derartige Individuen sind steril und kurzlebig.

| Dosis (ppm) | % Tiere mit Juvenoideffekten Beispiel 2 | Vergleich |
|---|---|---|
| 10 | 100 | ca. 60 |
| 4 | 100 | ca. 60 |
| 2 | 100 | ca. 60 |
| 1 | 100 | - |
| 0,4 | 100 | - |
| 0,2 | 100 | - |
| 0,1 | 100 | - |
| 0,04 | 100 | - |
| 0,02 | ca. 60 | - |
| 0,01 | ca. 60 | - |
| Kontrolle | 0 | - |

Beispiel B

Zuchtversuch mit Moskito-Larven (Aedes aegypti)

200 ml Leitungswasser werden mit der Wirkstoffaufbereitung versetzt und darauf mit 20 bis 30 Moskitolarven im 4. Larvenstadium besetzt. Die Gefäße stehen bei 25° C. Beobachtet werden Verpuppung und Schlüpfen der Imagines, welches nach 10 bis 12 Tagen erfolgt. Während der Beobachtungszeit wird einmal mit gepulvertem Zierfischfutter gefüttert.

| Dosis (ppm) | % Larven nicht geschlüpft Beispiel 2 | Vergleich |
|---|---|---|
| 0,4 | 100 | 100 |
| 0,2 | 100 | 100 |
| 0,1 | 100 | < 50 |
| 0,04 | 100 | 100 |
| 0,02 | 100 | 0 |
| 0,01 | 100 | 0 |
| 0,004 | < 50 | 0 |
| 0,002 | 100 | 0 |
| 0,001 | 0 | 0 |
| 0,0004 | 0 | 0 |
| Kontrolle | 0 | 0 |

Beispiel C

Entwicklungshemmung bei Prodenia litura
(Prüfung auf behandeltem Nährboden)

100 g des Standard-Nährbodens für Prodenia werden in 250 ml-Becher gefüllt und warm mit der

wäßrigen Wirkstoffaufbereitung sorgfältig gemischt. Nach Erkalten belegt man jedes Gefäß mit 5 Raupen im 3. Larvenstadium und lagert sie bei 23° C. Bonitiert werden typische Juvenoideffekte, nämlich die Rotfärbung der Raupen, der gestörte Bau einer Puppenwiege und die unterbleibende Verpuppung.

| Dosis | % Tiere mit Juvenoideffekten | |
|---|---|---|
| (ppm) | Beispiel 2 | Vergleich |
| 1,0 | 100 | 0 |
| 0,4 | 100 | 0 |
| 0,2 | ca. 60 | 0 |
| 0,1 | ca. 60 | 0 |
| 0,04 | 0 | 0 |
| 0,02 | 0 | 0 |
| 0,01 | 0 | 0 |
| Kontrolle | 0 | 0 |

## Ansprüche

1. Carboxamide der allgemeinen Formel I

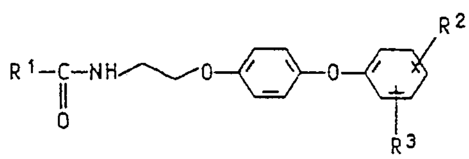

(I),

in der die Substituenten folgende Bedeutung haben:

$R^1$ $C_1$-$C_6$-Alkyl, $C_2$-$C_6$-Alkenyl, $C_2$-$C_6$-Alkinyl, $C_3$-$C_{10}$-Cycloalkyl, $C_3$-$C_{10}$-Cycloalkenyl oder $C_4$-$C_{10}$-Cycloalkenyl-alkyl und

$R^2$, $R^3$ Wasserstoff, Halogen, $C_1$-$C_3$-Alkyl, $C_1$-$C_3$-Alkoxy, $C_1$-$C_3$-Halogenalkyl, $C_1$-$C_3$-Halogenalkoxy oder Methylthio,

mit der Maßgabe, daß $R^1$ nicht Ethyl bedeutet, wenn $R^2$ und $R^3$ für Wasserstoff stehen.

2. Carboxamide der Formel I nach Anspruch 1, in der die Substituenten folgende Bedeutung haben:

$R^1$ $C_3$-$C_5$-Alkyl, $C_2$-$C_4$-Alkenyl, $C_2$-$C_4$-Alkinyl oder $C_3$-$C_6$-Cycloalkyl,

$R^2$ Wasserstoff, Fluor, Chlor, Brom, $C_1$-$C_3$-Alkyl, $C_1$-$C_3$-Alkoxy, $C_1$-$C_2$-Fluoralkyl oder $C_1$-$C_2$-Fluoralkoxy und

$R^3$ Wasserstoff, Fluor oder Chlor.

3. Carboxamide der Formel I nach Anspruch 1, in der die Substituenten folgende Bedeutung haben:

$R^1$ $C_3$-$C_5$-Alkyl, $C_2$-$C_4$-Alkenyl oder Cyclopropyl und

$R^2$, $R^3$ Wasserstoff.

4. Verfahren zur Herstellung von Carboxamiden der allgemeinen Formel I gemäß Anspruch 1, dadurch gekennzeichnet, daß man ein 2-Phenoxyethylamin der allgemeinen Formel II

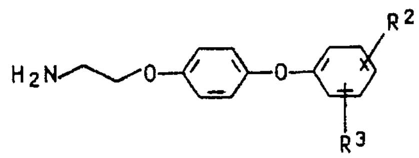

(II)

mit einem Säurehalogenid der allgemeinen Formel III

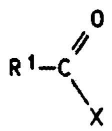

(III),

in der X für Halogen steht, umsetzt.

5. Schädlingsbekämpfungsmittel, enthaltend ein Carboxamid der allgemeinen Formel I

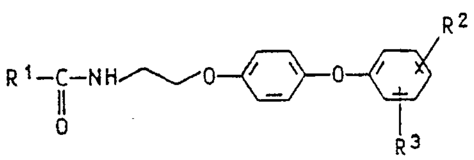

(I),

in der die Substituenten folgende Bedeutung haben:

$R^1$ $C_1$-$C_6$-Alkyl, $C_2C_6$-Alkenyl, $C_2$-$C_6$-Alkinyl, $C_3$-$C_{10}$-Cycloalkyl, $C_3$-$C_{10}$-Cycloalkenyl oder $C_4$-$C_{10}$-Cycloalkenyl-alkyl und

$R^2$, $R^3$ Wasserstoff, Halogen, $C_1$-$C_3$-Alkyl, $C_1$-$C_3$-Alkoxy, $C_1$-$C_3$-Halogenalkyl, $C_1$-$C_3$-Halogenalkoxy oder Methylthio,

neben hierfür üblichen Trägerstoffen.

6. Schädlingsbekämpfungsmittel nach Anspruch 5, dadurch gekennzeichnet, daß es 0,1 bis 95 Gew.-% eines Carboxamids der Formel I enthält.

7. Verfahren zur Bekämpfung von Schädlingen, dadurch gekennzeichnet, daß man die Schädlinge und/oder die von Schädlingen freizuhaltenden Flächen und/oder Räume mit einer für Schädlinge wirksamen Menge eines Carboxamids der Formel 1 gemäß Anspruch 5 behandelt.

8. Carboxamid der Formel I nach Anspruch 1, in der

$R^1$ Cyclopropyl und

$R^2$, $R^3$ Wasserstoff

bedeuten.

## Claims

1. A carboxamide of the formula I

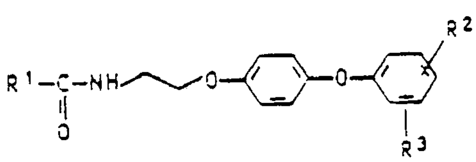

(I)

where $R^1$ is $C_1$-$C_6$-alkyl, $C_2$-$C_6$-alkenyl, $C_2$-$C_6$-alkynyl, $C_3$-$C_{10}$-cycloalkyl, $C_3$-$C_{10}$-cycloalkenyl or $C_4$-$C_{10}$-cyclo-alkenylalkyl, and $R^2$ and $R^3$ are each hydrogen, halogen, $C_1$-$C_3$-alkyl, $C_1$-$C_3$-alkoxy, $C_1$-$C_3$ haloalkyl, $C_1$-$C_3$-haloalkoxy or methylthio, with the proviso that $R^1$ is not ethyl when $R^2$ and $R^3$ are each hydrogen.

2. A carboxamide of the fomula I as claimed in claim 1, where $R^1$ is $C_3$-$C_5$-alXyl, $C_2$-$C_4$-alkenyl, $c_2$-$c_4$-alkynyl or $C_3$-$C_6$-cycloalkyl, $R^2$ is hydrogen, fluorine, chlorine, bromine, $C_1$-$C_3$-alkyl, $c_1$-$c_3$-alkoxy, $C_1$-$C_2$-fluoroalkyl or $C_1$-$C_2$-fluoroalkoxy, and $R^3$ is hydrogen, fluorine or chlorine.

3. A carboxamide of the formula I as claimed in claim 1, where $R^1$ is $C_3$-$C_5$-alkyl, $C_2$-$C_4$-alkenyl or cyclopropyl, and $R^2$ and $R^3$ are each hydrogen.

4. A process for the preparation of a carboxamide of the formula I as claimed in claim 1, which comprises reacting a 2-phenoxyethylamine of the formula II

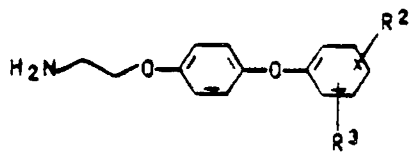

(II)

with an acyl halide of the formula III

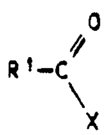

(III)

where X is halogen.

5. A pesticide which contains, in addition to the carriers customary for such substances, a carboxamide of the formula I

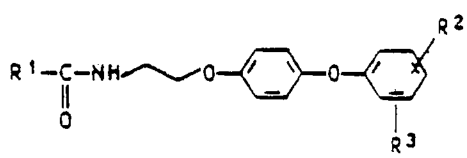

(I)

where $R^1$ is $C_1$-$C_6$-alkyl, $C_2$-$C_6$-alkenyl, $C_2$-$C_6$-alkynyl, $C_3$-$C_{10}$cycloalkyl, $C_3$-$C_{10}$-cycloalkenyl or $C_4$-$C_{10}$-cyclo-alkenylalkyl and $R^2$ and $R^3$ are each hydrogen, halogen, $C_1$-$C_3$alkyl, $C_1$-$C_3$-alkoxy, $C_1$-$C_3$-haloalkyl, $C_1$-$C_3$-haloalkoxy or methylthio.

6. A pesticide as claimed in claim 5, containing from 0.1 to 95 wt% of a carboxamide of the formula I.

7. A method of combating pests, wherein the pests and/or the areas and/or rooms to be kept free from pests are treated with an effective amount - for pests - of a carboxamide of the formula I as claimed in claim 5.

8. A carboxamide of the formula I as claimed in claim 1, where $R^1$ is cyclopropyl, and $R^2$ and $R^3$ are each hydrogen.

## Revendications

**1.** Carboxamides de formule générale I

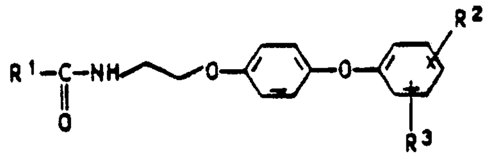

(I),

dans laquelle les substituants ont les significations suivantes :

- $R^1$ alkyle en $C_1$-$C_6$, alcényle en $C_2C_6$, alcynyle en $C_2$-$C_6$, cycloalkyle en C3-c10, cycloalcényle en $C_3$-$C_{10}$ ou cycloalcényl-$C_4$-$C_{10}$-alkyle et
- $R^2$, $R^3$ hydrogène, halogène, alkyle en $C_1$-$C_3$, alcoxy en $C_1$-$C_3$, halogéno-alkyle en $C_1$-$C_3$, halogéno-alcoxy en $C_1$-$C_3$ ou méthylthio,

étant entendu que $R^1$ ne représente pas éthyle si $R^2$ et $R^3$ sont mis pour hydrogène.

**2.** Carboxamides de formule I, selon la revendication 1, dans laquelle les substituants ont les significations suivantes :

- $R^1$ alkyle en $C_3$-$C_5$, alcényle en $C_2$-$C_4$, alcynyle en $C_2$-C4 ou cycloalkyle en $C_3$-$C_6$,
- $R^2$ hydrogène, fluor, chlore, brome, alkyle en $C_1$-$C_3$, alcoxy en $C_1$-$C_3$, fluoralkyle en $C_1$-$C_2$ ou fluoralcoxy en $C_1$-$C_2$ et
- $R^3$ hydrogène, fluor ou chlore.

**3.** Carboxamides de formule I, selon la revendication 1, dans laquelle les substituants ont les significations suivantes :

- $R^1$ alkyle en $C_3$-$C_5$, alcényle en $C_2$-$C_4$ ou cyclopropyle et
- $R^2$, $R^3$ hydrogène.

**4.** Procédé de préparation de carboxamides de formule générale I, selon la revendication 1, caractérisé par le fait que l'on fait réagir une 2-phénoxyéthylamine de formule générale II

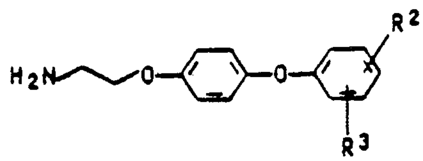

(II),

avec un halogénure d'acide de formule générale III

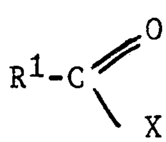

(III),

dans laquelle X est mis pour halogene.

**5.** Pesticides, contenant, outre des véhicules usuels, un carboxamide de formule générale I

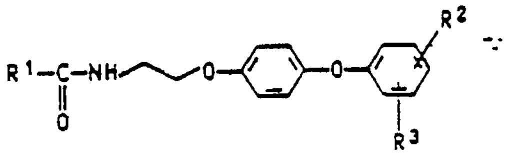

(I),

dans laquelle les substituants ont les significations suivantes :

- $R^1$ alkyle en $C_1$-$C_6$, alcényle en $C_2$-$C_6$, alcynyle en $C_2$-$C_6$, cycloalkyle en $C_3$-$C_{10}$, cycloalcényle en $C_3$-$C_{10}$ ou cycloalcényl-$C_4$-$C_{10}$-alkyle et
- $R^2$, $R^3$ hydrogène, halogène, alkyle en $C_1$-$C_3$, alcoxy en $C_1$-$C_3$, halogéno-alkyle en $C_1$-$C_3$, halogéno-alcoxy en $C_1$-$C_3$ ou méthylthio.

6. Pesticide selon la revendication 5, caractérisé par le fait qu'il renferme 0,1 à 95 % en poids d'un carboxamide de formule I.

7. Procédé de lutte contre les parasites, caractérisé par le fait que l'on traite les parasites et/ou les surfaces et/ou locaux à maintenir exempts de parasites avec une quantité efficace pour des parasites d'un carboxamide de formule I, selon la revendication 5.

8. Carboxamide de formule I, selon la revendication 1, dans laquelle
   - $R^1$ représente cyclopropyle et
   - $R^2$, $R^3$ , hydrogène.